# FASCICULE DE BREVET EUROPEEN

(11) **EP 1 583 575 B1**
(45) Date de publication et mention de la délivrance du brevet: **17.12.2008**
(21) Numéro de dépôt: 03795040.9
(22) Date de dépôt: 10.07.2003
(51) Int. Cl.: A61M 15/00

(54) **VALVE DE DISTRIBUTION DE PRODUIT FLUIDE ET DISPOSITIF DE DISTRIBUTION DE PRODUIT FLUIDE COMPORTANT UNE TELLE VALVE**
VENTIL ZUR ABGABE EINES FLÜSSIGEN PRODUKTSUND DIESES ENTHALTENDE VORRICHTUNG ZUR ABGABE EINES FLÜSSIGEN PRODUKTS
FLUID PRODUCT DISPENSING VALVE AND FLUID PRODUCT DISPENSING DEVICE COMPRISING SAME

(30) Priorité: 10.09.2002 FR 0211176
(43) Date de publication de la demande: 12.10.2005
(73) Titulaire: Valois SAS, 27110 Le Neubourg (FR)
(72) Inventeur: PARDONGE, Jean-Marc, 76520 Les Authieux-sur-le-Port-Saint-Ouen (FR)
(74) Mandataire: CAPRI
(86) Numéro de dépôt international: PCT/FR2003/002180
(87) Numéro de publication internationale: WO 2004/024220

(56) Documents cités:
- EP-A- 0 308 524
- EP-A- 0 534 749
- WO-A-01/89616
- WO-A-01/96210
- WO-A-02/49569
- FR-A- 1 340 404
- JP-A- 53 096 516
- US-A- 3 184 115
- US-A- 3 236 420
- US-A- 3 292 823

## Description

La présente invention concerne une valve de distribution de produit fluide, ainsi qu'un dispositif de distribution de produit fluide comportant une telle valve.

Les valves de distribution de produit fluide sont bien connues dans l'état de la technique. Elles comportent généralement un corps de valve dans lequel une soupape coulisse entre une position de repos et une position de distribution, dans laquelle le produit est distribué. Le produit est généralement distribué au moyen d'un gaz propulseur, et ce type de valve est communément appelé « valve aérosol ». Dans le cas des valves doseuses, le corps de valve comporte une chambre de valve qui définit de manière précise le volume de produit fluide distribué à chaque actionnement de la valve.

De manière classique, les valves doseuses distribuent généralement des volumes de dose situés entre 50 et 100 µl. Dans ce cas, la durée de la distribution d'une dose est relativement faible. Par contre, dans les valves doseuses destinées à distribuer des volumes nettement plus importants, par exemple supérieurs à 500 µl, la nature même du produit aérosol peut engendrer des problèmes de fonctionnement de la valve. Ainsi, la durée de distribution d'une dose étant relativement importante, ces valves qui dispensent de très grandes doses se trouvent soumises à des refroidissements intenses dus à la nature du produit aérosol à distribuer et au mode de distribution. En particulier, la détente du gaz propulseur provoque un tel refroidissement. Ces refroidissements peuvent dans certains cas entraîner un dysfonctionnement de la valve, notamment en bouchant les canaux de sortie, particulièrement au niveau de la soupape.

Les documents WO 01/89616, WO 02/49562, US-3 292 823 et WO 01/96210 décrivent des dispositifs de l'art antérieur.

La présente invention a pour but de fournir une valve de distribution de produit fluide qui ne reproduit pas les inconvénients susmentionnés.

Plus particulièrement, la présente invention a pour but de fournir une valve de distribution de produit fluide qui fonctionne de manière sûre et fiable, indépendamment du volume de produit fluide distribué lors de son actionnement.

La présente invention a également pour but de fournir une telle valve de distribution de produit fluide qui soit simple et peu coûteuse à fabriquer et à assembler.

La présente invention a également pour but de fournir un dispositif de distribution de produit fluide comportant une telle valve.

La présente invention a donc pour objet une valve de distribution de produit fluide telle que décrite dans la revendication 1. Des modes de réalisation avantageux sont décrits dans les revendications dépendantes.

La présente invention a également pour objet un dispositif de distribution de produit fluide comportant un réservoir de produit fluide et une valve telle que définie ci-dessus.

Avantageusement, le dispositif de distribution de produit fluide comporte une tête de distribution montée sur la soupape de ladite valve.

Avantageusement, ladite tête de distribution, en particulier la partie coopérant avec la soupape, comporte lesdits moyens de régulation thermique.

D'autres caractéristiques et avantages de la présente invention apparaîtront plus clairement au cours de la description détaillée suivante, faite en référence aux dessins joints, donnés à titres d'exemples non limitatifs, et sur lesquels
- la figure 1 est une vue schématique en section transversale d'un dispositif de distribution de produit fluide comportant une valve de distribution selon un premier mode de réalisation de la présente invention ;
- la figure 2 est une vue similaire à celle de la figure 1, représentant un second mode de réalisation de la présente invention ; et
- la figure 3 est une vue similaire à celles des figures 1 et 2, montrant un troisième mode de réalisation de la présente invention.

En référence aux figures, un dispositif de distribution de produit fluide comporte un réservoir 1 sur lequel est montée une valve 10 au moyen d'une bague 2, de préférence une bague ou capsule sertissable comme représenté sur les figures. Un joint de col 3 est généralement interposé entre le col du réservoir 1 et ladite bague 2. Le réservoir 1 contient un produit fluide dont une partie est distribuée à chaque actionnement de la valve 10.

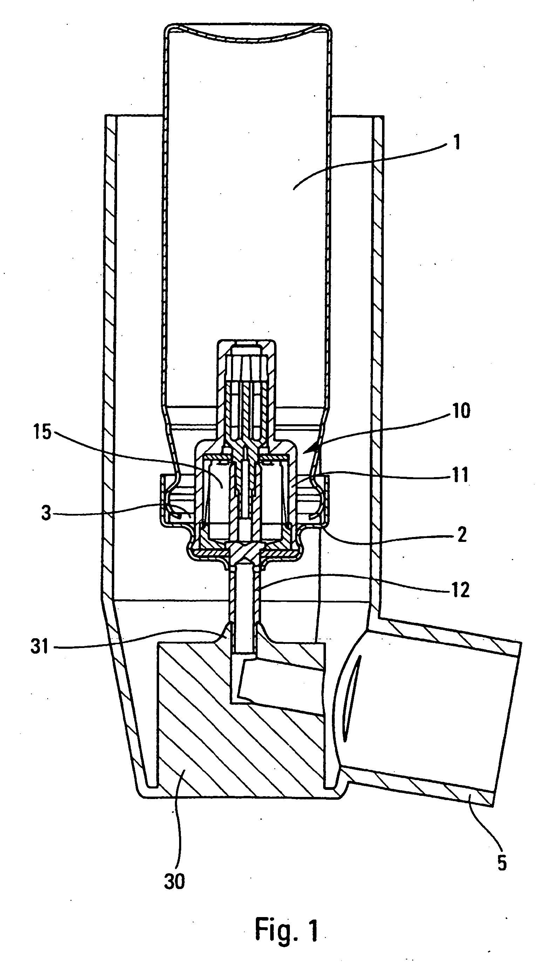

De préférence, cette valve est une valve doseuse, et comporte un corps de valve 11 renfermant une chambre de valve 15 qui définit le volume de chaque dose de produit distribué à chaque actionnement de la valve 10. Une soupape 12 est montée coulissante dans ledit corps de valve 11 entre une position de repos, représentée sur les figures, et une position d'actionnement, dans laquelle ladite soupape 12 est enfoncée à l'intérieur dudit corps de valve 11 pour distribuer la dose de produit fluide contenu dans la chambre de valve 15. Le produit fluide est distribué au moyen d'un gaz propulseur, de préférence du type HFA-134a ou HFA-227, qui sont des gaz non nocifs pour l'environnement. Une tête de distribution 30 est généralement assemblée sur la soupape 12 pour relier l'orifice de sortie de ladite soupape 12 avec un orifice de distribution du dispositif de distribution de produit fluide. Dans l'exemple représenté sur les figures, la tête de distribution 30 relie la soupape 12 à un embout buccal 5 du dispositif. Bien entendu, la présente invention ne se limite pas à l'exemple de réalisation représenté sur les figures 1 à 3, et les différentes parties constitutives du dispositif (réservoir, valve, bague de fixation, tête de distribution, corps du dispositif, etc) peuvent être réalisées d'une manière quelconque appropriée.

Selon l'invention, la valve 10 comporte des moyens de régulation thermique pour limiter le refroidissement de la soupape 12 lors de l'actionnement de la valve 10, et donc de la distribution du produit fluide. Comme expliqué précédemment, le problème d'un refroidissement intense se pose surtout avec des valves destinées à distribuer des gros volumes à chaque actionnement, par exemple pouvant aller jusqu'à 600 µl. Toutefois, la présente invention ne se limite pas exclusivement à des valves distribuant ces volumes importants, mais plus généralement à toutes les valves dans lesquelles se pose un problème de fonctionnement et de fiabilité de la valve du à un refroidissement important lors de la distribution.

En référence à la figure 1, il est représenté un premier mode de réalisation de la présente invention. Dans ce premier mode de réalisation, la soupape 12 est réalisée partiellement en un matériau ayant un fort coefficient de conduction thermique. Ce matériau peut être un métal, et notamment de l'aluminium. Bien entendu, tout matériau peut être un métal, et notamment de l'aluminium. Bien entendu, tout autre matériau ayant un fort coefficient de conduction thermique pourrait être utilisé. Le fait de réaliser la soupape 12 avec un tel matériau conducteur thermique permet d'augmenter le temps de refroidissement, et donc de limiter ce refroidissement. On évite ainsi que les canaux de sortie de la valve, et notamment de la soupape, ne se bouchent pendant cette distribution.

Selon l'invention, la soupape 12 est réalisée seulement partiellement en matériau conducteur thermique, tel que l'aluminium. La soupape est réalisée en matériau conducteur thermique seulement sur sa partie extérieure s'étendant hors du corps de valve. Cette partie extérieure est surmoulée sur une partie intérieure de la soupape, coulissant à l'intérieur du corps de valve, et est réalisée en un matériau synthétique classique. Le surmoulage garantit une parfaite étanchéité. Cette mise en oeuvre permet de limiter le contact du produit actif avec le métal, et s'adapte facilement sur les soupapes existantes.

De manière complémentaire, la tête 30 aussi pourrait être réalisée en un matériau conducteur thermique, par exemple de l'aluminium. Avantageusement, lorsque la tête 30 est réalisée d'une pièce avec le corps du distributeur, le fait de réaliser cette pièce en matériau conducteur thermique a pour effet de transmettre la chaleur de la main de l'utilisateur lorsque celui-ci tient le distributeur. Ceci permet encore davantage de limiter le refroidissement de la soupape.

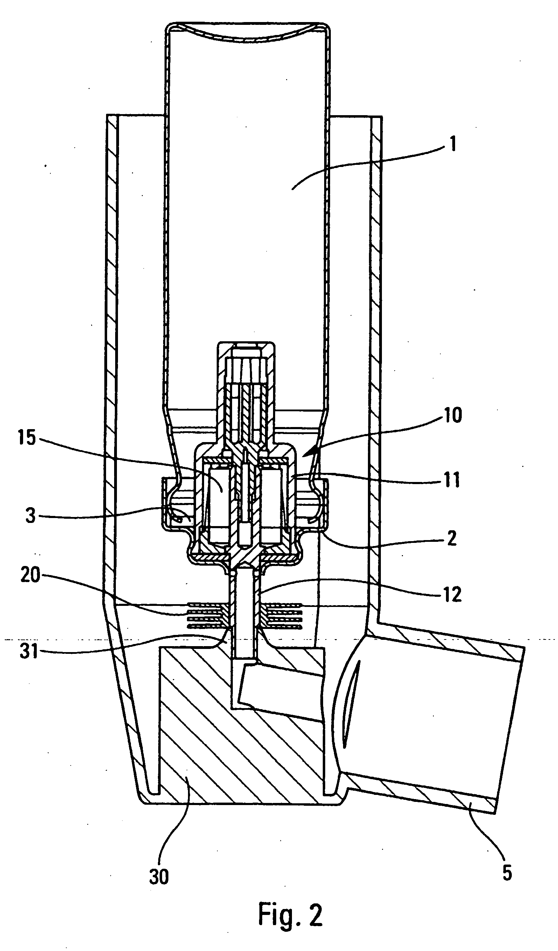

La figure 2 montre un autre mode de réalisation de la présente invention, dans lequel les moyens de régulation thermique comportent en plus des ailettes de refroidissement 20. Dans l'exemple de la figure 2, ces ailettes de refroidissement 20 sont disposées autour de la soupape 12 et s'étendent environ transversalement à l'axe centrale de ladite soupape 12. La figure 2 montre quatre ailettes s'étendant environ parallèlement les unes aux autres, chaque ailette étant formée par un disque comportant un trou central à travers lequel passe la soupape 12. Bien entendu, ces ailettes de refroidissement 12 peuvent être d'un nombre quelconque souhaité, et leur forme peut également être différente, l'objectif étant d'obtenir une surface importante afin de permettre une conduction thermique importante suffisante pour limiter le refroidissement et ainsi empêcher un dysfonctionnement de la valve. Les ailettes de refroidissement 20 peuvent également être réalisées en un matériau plastique. En variante, pour augmenter encore davantage la conduction thermique, les ailettes de refroidissement 20 peuvent être réalisées en un matériau à fort coefficient de conduction thermique, par exemple un métal, et notamment de l'aluminium.

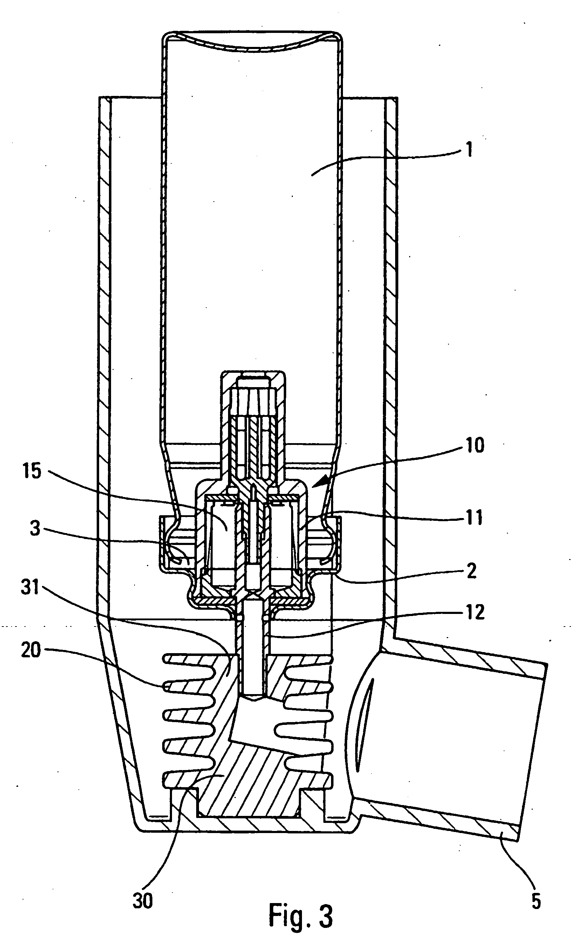

La figure 3 représente un autre mode de réalisation de la présente invention. Dans ce troisième mode de réalisation, les ailettes de refroidissement 20 ne sont pas formées sur la soupape 12, mais dans la tête de distribution 30 qui coopère avec ladite soupape 12. C'est plus particulièrement au niveau de la partie 31 qui est assemblée sur la soupape 12 qu'il est avantageux de former des moyens de régulation thermique, par exemple les ailettes de refroidissement 20. Bien entendu, ces ailettes de refroidissement 20 peuvent être d'un nombre quelconque et s'étendre sur la totalité de ladite tête 30, comme représenté sur la figure 3.

Bien entendu, il est tout à fait envisageable de combiner les différents modes de réalisation décrits et donc d'utiliser une soupape partiellement conductrice thermique ainsi que des moyens de régulation thermique 20 à la fois autour de la soupape 12 et dans la tête de distribution 30, si nécessaire. L'objectif essentiel de la présente invention est de limiter le refroidissement lors de la distribution du produit pour éviter un dysfonctionnement de la valve, notamment par bouchage, ce qui peut se produire lorsque la valve distribue des très grands volumes, tel qu'expliqué précédemment.

Bien que décrite en référence à des exemples de réalisation particuliers représentés sur les figures, il est entendu que la présente invention ne se limite pas aux modes de réalisation représentés et décrits ci-dessus, mais qu'elle englobe au contraire toutes modifications qui pourraient y être apportées par un homme du métier, sans sortir du cadre défini par les revendications annexées. Par exemple, il serait envisageable de prévoir des moyens de chauffage ou similaire au niveau ou à proximité de la soupape. D'autres types de moyens de régulation thermique sont aussi envisageables.

## Revendications

1. Valve (10) de distribution de produit fluide comportant un corps de valve (11) et une soupape(12) coulissant dans ledit corps de valve (11) entre une position de repos et une position de distribution, **caractérisée en ce que** ladite valve (10) comporte des moyens de régulation thermique (12, 20) pour limiter le refroidissement de la soupape (12) lors de la distribution du produit, lesdits moyens de régulation thermique comprenant une soupape (12) réalisée partiellement en un matériau conducteur thermique, ladite soupape (12) comportant une partie intérieure, coulissant à l'intérieur du corps de valve (11) réalisée en un premier matériau synthétique, et une partie extérieure, s'étendant au moins partiellement à l'extérieur du corps de valve, réalisée en un second matériau qui est conducteur thermique, lesdites parties intérieure et extérieure étant fixées l'une à l'autre par surmoulage.

2. Valve selon la revendication 1, dans laquelle lesdits moyens de régulation thermique comprennent en outre une tête (30) coopérant avec ladite soupape (12), ladite tête (30) étant réalisée en matériau conducteur thermique.

3. Valve selon l'une quelconque des revendications précédentes, dans laquelle lesdits moyens de régulation thermique comprennent en outre des ailettes de refroidissement (20) coopérant avec ladite soupape (12).

4. Valve selon la revendication 3, dans laquelle lesdites ailettes (20) sont disposées, autour de ladite soupape (12).

5. Valve selon la revendication 3, dans laquelle lesdites ailettes (20) sont disposées dans une tête (30) coopérant avec ladite soupape (12).

6. Valve selon l'une quelconque des revendications 3 à 5, dans laquelle lesdites ailettes (20) s'étendent environ parallèlement les unes aux autres, et sensiblement transversalement à l'axe centrale de ladite soupape (12).

7. Valve selon l'une quelconque des revendications 3 à 6, dans laquelle lesdites ailettes (20) sont réalisées en un matériau conducteur thermique.

8. Valve selon l'une quelconque des revendications précédentes, dans laquelle ledit matériau conducteur thermique est métallique, notamment de l'aluminium.

9. Valve selon l'une quelconque des revendications précédentes, fonctionnant avec un gaz propulseur pour distribuer le produit fluide.

10. Valve selon la revendication 9, dans laquelle ledit gaz propulseur comprend des gaz du type HFA-134a ou Ha-227.

11. Valve selon l'une quelconque des revendications précédentes, dans laquelle ladite valve (10) est une valve doseuse, ledit corps de valve (11) comportant une chambre de valve (15) définissant un volume de produit fluide à distribuer à chaque actionnement de la valve (10).

12. Valve selon la revendication 11, dans laquelle ledit volume de produit fluide distribué à chaque actionnement est supérieur à 500 µl.

13. Dispositif de distribution de produit fluide, comportant un réservoir (1) de produit fluide, **caractérisé en ce qu'**il comporte une valve (10) selon l'une quelconque des revendications 1 à 12.

14. Dispositif selon la revendication 13, comportant une tête de distribution (30) montée sur la soupape (12) de ladite valve (10).

15. Dispositif selon la revendication 14, dans lequel ladite tête de distribution (30), en particulier la partie (31) coopérant avec la soupape (12), comporte lesdits moyens de régulation thermique (20).

## Claims

1. A fluid-dispenser valve (10) comprising a valve body (11), and a valve member (12) slidable in said valve body (11) between a rest position and a dispensing position, said valve (10) being **characterized in that** it includes temperature regulator means (12, 20) for limiting cooling of the valve member (12) while the fluid is being dispensed, said temperature regulator means comprising a valve member (12) made, in part of a thermally-conductive material, and said valve member (12) including an inner portion, slidable inside the valve body (11), and made of a first synthetic material, and an outer portion, extending, at least in part, outside the valve body, and made of a second material that is thermally conductive, said inner and outer portions being secured to each other by overmolding.

2. A valve according to claim 1, in which said temperature regulator means further comprise a head (30) co-operating with said valve member (12), said head (30) being made of a thermally-conductive material.

3. A valve according to any preceding claim, in which said temperature regulator means further comprise cooling plates (20) co-operating with said valve member (12).

4. A valve according to claim 3, in which said plates (20) are disposed around said valve member (12).

5. A valve according to claim 3, in which said plates (20) are disposed in a head (30) co-operating with said valve member (12).

6. A valve according to any one of claims 3 to 5, in which said plates (20) extend approximately parallel to one another, and substantially transversely to the central axis of said valve member (12).

7. A valve according to any one of claims 3 to 6, in which said plates (20) are made of a thermally-conductive material.

8. A valve according to any preceding claim, in which said thermally-conductive material is a metal, in particular aluminum.

9. A valve according to any preceding claim, operating with a propellant gas so as to dispense the fluid.

10. A valve according to claim 9, in which said propellant gas comprises gases of the HFA-134a or HFA-227 type.

11. A valve according to any preceding claim, in which said valve (10) is a metering valve, said valve body (11) including a valve chamber (15) defining a volume of fluid to be dispensed each time the valve (10) is actuated.

12. A valve according to claim 11, in which said volume of fluid dispensed at each actuation is greater than 500 µl.

13. A fluid dispenser device comprising a fluid reservoir (1), said device being **characterized in that** it further comprises a valve (10) according to any one of claims 1 to 12.

14. A device according to claim 13, including a dispenser head (30) mounted on the valve member (12) of said valve (10).

15. A device according to claim 14, in which said dispenser head (30), and in particular the portion (31) co-operating with the valve member (12), includes said temperature regulator means (20).

## Patentansprüche

1. Abgabeventil (10) für ein fluidförmiges Produkt, das einen Ventilkörper (11) und ein Ventilelement (12) umfasst, das in dem Ventilkörper (11) zwischen einer Ruhelage und einer Abgabestellung gleitet, **dadurch gekennzeichnet, dass** das Ventil (10) eine thermische Regulierungsvorrichtung (12, 20) umfasst, um das Abkühlen des Ventilelementes (12) während der Abgabe des Produktes zu begrenzen, wobei thermische Regulierungsvorrichtung ein Ventilelement (12) umfasst, das teilweise aus einem Wärmeleitenden Material besteht, wobei das Ventilelement (12) einen inneren Teil, der im Inneren des Ventilkörpers (11) gleitet und aus einem ersten synthetischen Material hergestellt ist, und einen äußeren Teil umfasst, der sich zumindest teilweise außerhalb des Ventilkörpers erstreckt und aus einem zweiten Material hergestellt ist, das wärmeleitfähig ist, wobei der innere und der äußere Teil aneinander durch Überformen befestigt sind.

2. Ventil nach Anspruch 1, bei dem die thermische Regulierungsvorrichtung weiterhin einen Kopf (30) umfasst, der mit dem Ventilelement (12) zusammenwirkt, wobei der Kopf (30) aus einem Wärmeleitenden Material hergestellt ist.

3. Ventil nach einem der vorhergehenden Ansprüche, bei dem die thermische Regulierungsvorrichtung weiterhin Kühlrippen (20) umfasst, die mit dem Ventilelement (12) zusammenwirken.

4. Ventil nach Anspruch 3, bei welchem die Rippen (20) um das Ventilelement (12) herum angeordnet sind.

5. Ventil nach Anspruch 3, bei welchem die Rippen (20) in einem Kopf (30) angeordnet sind, der mit dem Ventilelement (12) zusammenwirkt.

6. Ventil nach einem der Ansprüche 3 bis 5, bei welchem die Rippen (20) sich ungefähr parallel zu einander und im Wesentlichen quer zur zentralen Achse des Ventilelements (12) erstrecken.

7. Ventil nach einem der Ansprüche 3 bis 6, bei welchem die Rippen (20) aus einem Wärmeleitenden Material hergestellt sind.

8. Ventil nach einem der vorhergehenden Ansprüche, bei welchem das Wärmeleitende Material metallisch, insbesondere Aluminium ist.

9. Ventil nach einem der vorhergehenden Ansprüche, das mit einem Treibgas arbeitet, um das fluidförmige Produkt abzugeben.

10. Ventil nach Anspruch 9, bei welchem das Treibgas Gas vom Typ HFA-134a oder HFA-227 umfasst.

11. Ventil nach einem der vorhergehenden Ansprüche, bei welchem das Ventil (10) ein Dosierventil ist, wobei der Ventilkörper (11) eine Ventilkammer (15) umfasst, die das Volumen für das bei jeder Betätigung des Ventils (10) abzugebende fluidförmige Produkt definiert.

12. Ventil nach Anspruch 11, bei welchem das bei jeder Betätigung abzugebende Volumen des fluidförmigen Produktes mehr als 500 µl ist.

13. Abgabevorrichtung für ein fluidförmiges Produkt, die einen Behälter (1) für das fluidförmige Produkt umfasst, **dadurch gekennzeichnet, dass** sie weiterhin ein Ventil (10) nach einem der Ansprüche 1 bis 12 umfasst.

14. Vorrichtung nach Anspruch 13, die einen Abgabekopf (30) umfasst, der auf dem Ventilelement (12) des Ventils (10) montiert ist.

15. Vorrichtung nach Anspruch 14, bei welchem der Abgabekopf (30), insbesondere sein Teil (31), der mit dem Ventilelement (12) zusammenarbeitet, besagte thermische Regulierungsvorrichtung (20) umfasst.
